# EUROPÄISCHE PATENTSCHRIFT

(11) **EP 3 115 037 B1**
(45) Veröffentlichungstag und Bekanntmachung des Hinweises auf die Patenterteilung: **15.08.2018**
(21) Anmeldenummer: 15175806.7
(22) Anmeldetag: 08.07.2015
(51) Int. Cl.: A61K 9/02, A61K 31/58

(54) **PHARMAZEUTISCHE FORMULIERUNG ZUR BEHANDLUNG VON ENTZÜNDLICHEN VERÄNDERUNGEN DES ENDDARMS**
PHARMACEUTICAL PREPARATIONS FOR THE TREATMENT OF INFLAMMATORY CONDITIONS OF THE RECTUM
FORMULATION PHARMACEUTIQUE POUR LE TRAITEMENT DE MODIFICATIONS INFLAMMATOIRES DE RECTUM

(43) Veröffentlichungstag der Anmeldung: 11.01.2017
(73) Patentinhaber: Dr. Falk Pharma GmbH, 79108 Freiburg (DE)
(72) Erfinder: Wilhelm, Rudolph, Dr., 76476 Bischweier (DE); Pröls, Markus, Dr., 79100 Freiburg/Breisgau (DE); Greinwald, Roland, Dr., 79341 Kenzingen (DE); Mohrbacher, Ralf, 79111 Freiburg (DE)
(74) Vertreter: Keller, Günter

(56) Entgegenhaltungen:
- WO-A2-2008/156671
- DE-A1- 19 849 737
- DE-U1- 29 717 252

## Beschreibung

Als Crohn's Disease sowie als ulzerative Colitis werden chronische entzündliche Erkrankungen des Gastrointestinaltrakts bezeichnet. Die Ethiologie dieser Erkrankungen ist unbekannt, wenngleich ein Autoimmunaspekt dieser Erkrankungen vielfach angenommen wird. Die Erkrankung "Crohn's Disease" kann eine Vielzahl von klinischen Erscheinungsformen beinhalten und diese Erkrankung kann an verschiedenen Teilen des Dünndarms sowie auch des Dickdarms lokalisiert werden. Die ulzerative Colitis ist eine entzündliche Darmerkrankung, die sich im Wesentlichen auf den Dickdarm beschränkt. Die Erkrankung ist charakterisiert durch wiederkehrende Entzündungszustände, die in erster Linie die Mukosa-Schicht und gelegentlich auch die Submukosa-Schicht des Kolons befallen. Akute Entzündungszustände sind durch chronischen Durchfall oder Verstopfung, Blutungen im Darmbereich, Krämpfe und Bauchschmerzen charakterisiert.

Gelegentlich wird hiervon die ulzerative Proktitis unterschieden, die als eine mildere Form der ulzerativen Colitis angesehen wird. Zur Therapie dieser Erkrankungen können speziell formulierte oral einnehmbare Formulierungen verwendet werden. Aufgrund des mit der Erkrankung häufig einhergehenden Durchfalls kann aber die orale Verabreichung nachteilig sein.

Bekannt sind im Stand der Technik auch rektal verabreichbare pharmazeutische Formulierungen. Gross et al., Aliment.Pharmacol.Ther. 2006, 23, 303-312 haben bei der Behandlung von aktiver ulzerativer Proctitis oder Proctosigmoiditis Budesonid-Schäume mit Budesonid-Klysmen verglichen.

Belluzzi et al. (Gastroenterology, Vol. 104 (4, Suppl.) 1993) haben Suppositorien mit 5-Aminosalicylsäure oder Budesonid vorgestellt. Die dort verwendeten Budesonidzäpfchen enthielten 0,5 mg Budesonid und wurden dreimal täglich verabreicht. Alternativ wurden Suppositorien mit 500 mg 5-Aminosalicylsäure dreimal täglich verabreicht.

US 5,449,520 offenbart pharmazeutische Zusammensetzungen zur rektalen Verabreichung, die ein am Kolon topisch wirkendes Medikament enthalten. Die dort offenbarten Rektalschäume enthalten als pharmazeutischen Wirkstoff Mesalazin oder Budesonid. Die WO 2015/073846 beschreibt ein Verfahren zur Behandlung von ulzerativer Colitis, bei dem ein Rektalschaum zum Einsatz kommt. Dieser Schaum ist eine Emulsion enthaltend Budesonid, Propylenglykol, Cetylalkohol, Wasser und geeignete Zusatzstoffe.

Nachteilig bei den bekannten pharmazeutischen Formulierungen ist einerseits, dass manche Patienten Klysmen oder auch Rektalschäume nicht gerne verwenden. Ein anderes Erfordernis ist, dass eine pharmazeutische Formulierung wünschenswert ist, die den Wirkstoff Budesonid in einer lagerstabilen Form bereitstellt.

Es ist eine Aufgabe der vorliegenden Erfindung, eine pharmazeutische Formulierung von Budesonid zur rektalen Applikation zur Verfügung zu stellen, die lagerstabil ist und die eine durch optimale Form, Konsistenz und geeignete Größe schmerzfreie Einführung sowie eine gezielte, lokale und auf den Enddarm begrenzte Applikation des Wirkstoffes ermöglicht.

Diese Kriterien werden erfindungsgemäß durch die Darreichungsform der Zäpfchen, auch Suppositorien genannt, erfüllt. Suppositorien enthalten eine Einzeldosis des arzneilich wirksamen Bestandteils Budesonid, die in einer lipidhaltigen oder wasserlöslichen Zubereitung gelöst (Lösungs-Zubereitung), emulgiert (Emulsions-Zubereitung) und suspendiert (Suspensions-Zubereitung) sein kann.

Die erfindungsgemäßen pharmazeutischen Formulierungen sind in hohem Maße lagerstabil. Ein Problem bei der Bereitstellung von Suppositorien (Zäpfchen) ist, dass, wenn derartige Präparate bei Raumtemperatur oder geringfügig erhöhter Temperatur (20-30°C), die gerade während der Sommermonate oder in wärmeren Gegenden häufig auftritt, gelagert werden, der pharmakologisch aktive Wirkstoff Budesonid abgebaut wird zu biologisch nicht aktiven oder weniger aktiven Abbauprodukten. Die erfindungsgemäßen Formulierungen sind über einen längeren Zeitraum (12-24 Monate) auch bei erhöhter Temperatur (20-30°C) lagerstabil. Dies bedeutet, dass nach einer Lagerung über 24 Monate bei 25°C noch wenigstens 90 %, bevorzugt wenigstens 95 % und bevorzugt wenigstens 97 % des ursprünglich eingesetzten Wirkstoffes (Budesonid) in pharmakologisch aktiver Form vorliegen.

Die vorliegende Erfindung betrifft eine Zubereitung zur rektalen Anwendung, insbesondere ein Suppositorium, das als therapeutisch wirksamen Bestandteil Budesonid oder ein pharmazeutisch verträgliches Salz oder Derivat davon umfasst und nach Einführen zur Behandlung von entzündlichen Erkrankungen des Rektums (Enddarmentzündung) angewendet wird. Dabei müssen hohe Anforderungen an die Auswahl einer geeigneten Grundlage gestellt werden. Diese Grundlage muss sowohl chemisch stabil und inert sein und damit eine hohe Verträglichkeit mit dem arzneilich wirksamen Bestandteil aufweisen, als auch schleimhautverträglich und damit frei von Schleimhautreizungen bei der Anwendung sein. Weiterhin muss sie nach der Applikation durch Schmelzen oder Auflösen den eingearbeiteten arzneilich wirksamen Bestandteil am Ort der Anwendung zuverlässig freigeben.

Gegenstand der vorliegenden Erfindung ist daher eine lagerstabile pharmazeutische Formulierung zur rektalen Verabreichung, die Budesonid oder ein pharmazeutisch verträgliches Salz oder Derivat davon und wenigstens 80 Gew.-% eines Hartfettes oder eines Gemisches verschiedener Hartfette bezogen auf das Gesamtgewicht der Formulierung sowie wenigstens ein damit verträgliches Antioxidationsmittel enthält gemäß Anspruch 1. In einer bevorzugten Ausführungsform der vorliegenden Erfindung wird eine Hartfettzusammensetzung gewählt, die einen kleinen Abstand zwischen dem Schmelz- und Erstarrungspunkt aufweist. Bei dem Schmelzpunkt handelt es sich um die Temperatur, bei der das Suppositorium schmilzt. Dieser Schmelzpunkt liegt bevorzugt zwischen etwa 33,5°C und etwa 35,5°C, bevorzugt zwischen 34,0°C und 35,0°C. Der Erstarrungspunkt ist diejenige Temperatur, bei der das Suppositorium nach der Herstellung erstarrt, also der Punkt, bei dem das Suppositorium nach der Herstellung fest wird. Dieser Erstarrungspunkt liegt erfindungsgemäß bevorzugt zwischen etwa 32,5°C und 34,5°C, besonders bevorzugt bei 33,0°C bis 34,0°C.

In einer bevorzugten Ausführungsform hat das erfindungsgemäß eingesetzte Hartfett einen hohen Anteil von Triglyceriden, der bevorzugt über 80 Gew.-%, besonders bevorzugt über 90 Gew.-% und ganz besonders bevorzugt über 95 Gew.-% liegt.

Als Kennzahl dieser Hartfette ist die sogenannte Hydroxylzahl bekannt. Erfindungsgemäß haben die eingesetzten Hartfette eine niedrige Hydroxylzahl, die im Bereich von 1 bis 15, bevorzugt 5 bis 15 und besonders bevorzugt 5 bis 10 liegt.

Eine weitere Eigenschaft der bevorzugten Hartfette ist, dass der Anteil der ungesättigten Fettsäuren weniger als 1 Gew.-%, besonders bevorzugt weniger als 0,5 Gew.-% beträgt.

Die erfindungsgemäßen Suppositorien sind für die anale Verabreichung konzipiert. Daher ist die Form so gewählt, dass sie bequem appliziert werden können und von einem Großteil der Patienten als gut annehmbar angesehen werden, weil sie weder Schmerzen noch ein unangenehmes Gefühl bei der Verabreichung hervorrufen. In bevorzugter Ausführungsform haben die Suppositorien eine sogenannte "Torpedoform". Wichtig ist auch die Größe der Suppositorien, die durch das Gesamtgewicht der Suppositorien bestimmt wird. Bevorzugt liegt das Gewicht zwischen 0,8 g und 1,2 g, besonders bevorzugt zwischen 0,95 g und 1,05 g.

In einer bevorzugten Ausführungsform beinhaltet das erfindungsgemäße Suppositorium als Wirkstoff Budesonid in einer Menge zwischen 1,8 mg und 2,2 mg pro Suppositorium, bevorzugt zwischen 1,9 mg und 2,1 mg Budesonid pro Suppositorium und ganz besonders bevorzugt zwischen 1,95 mg und 2,05 mg Budesonid pro Suppositorium.

In einer anderen bevorzugten Ausführungsform beinhaltet das erfindungsgemäße Suppositorium als Wirkstoff Budesonid in einer Menge zwischen 3,8 mg und 4,2 mg pro Suppositorium, bevorzugt zwischen 3,9 mg und 4,1 mg Budesonid pro Suppositorium und ganz besonders bevorzugt zwischen 3,95 mg und 4,05 mg Budesonid pro Suppositorium.

In bevorzugter Ausführungsform beinhalten die erfindungsgemäßen Suppositorien als Antioxidationsmittel Ascorbylpalmitat. Die Konzentration des Ascorbylpalmitats beträgt bevorzugt 50 ppm bis 200 ppm, besonders bevorzugt 125 bis 175 ppm und ganz besonders bevorzugt 150 ppm.

Ein weiterer bevorzugter Aspekt der vorliegenden Erfindung ist, dass das Budesonid in mikronisierter Form vorliegt. Mikronisierte Form bedeutet, dass die Partikelgröße des Wirkstoffes sehr klein ist, wobei 100 % der Partikel kleiner als 10 µm pro Partikel sind.

Ein wesentlicher Aspekt der vorliegenden Erfindung ist die Lagerstabilität der erfindungsgemäßen Suppositorien. Durch verschiedene Verfahrensschritte während der Herstellung kann die Lagerstabilität weiter erhöht werden. Einerseits wird die Herstellung der Suppositorien unter Ausschluss von Sauerstoff durchgeführt. Dies kann entweder durch eine Stickstoffbegasung während der Herstellung oder Arbeiten unter Edelgasatmosphäre bewirkt werden.

Andererseits wird bei der Herstellung der Suppositorien die aufgeschmolzene Masse bevorzugt in eine Blisterfolie gegossen und dort erfolgt die Erhärtung. In einer bevorzugten Ausführungsform sind die erfindungsgemäßen Suppositorien in einer gasundurchlässigen Folie verpackt.

Allgemein bekannt ist die Verwendung von lipidhaltigen oder wasserlöslichen Zubereitungen als Grundlage für Suppositorien. Bevorzugt werden als Lipide Triglyceride verwendet. Hartfett ist ein halbsynthetisches Gemisch aus Mono-, Di- und Triglyceriden gesättigter Fettsäuren. Beginnend mit Palmkern- und Kokosfetten können nach Verseifung und erneuter Veresterung von Glycerin mit geeigneten gesättigten Fettsäuren definierte Hartfette mit bestimmten Schmelzeigenschaften und bestimmten Hydroxylzahlen über das Verhältnis von Mono-, Di- und Triglyceriden erhalten werden. Über die Auswahl an Fettsäuren und den Veresterungsgrad lassen sich somit die Eigenschaften von Hartfett modifizieren und Eigenschaften wie Schmelzbereich, Wasseraufnahmefähigkeit und Sprödigkeit beeinflussen. Durch das Fehlen von ungesättigen Fettsäuren haben Hartfette bessere Stabilitätseigenschaften als Kakaobutter, die als Suppositoriengrundlage daher nur noch eine untergeordnete Rolle spielt.

Die erfindungsgemäß bevorzugt verwendeten Hartfette basieren auf Glyceriden von gesättigten C₁₂-C₁₈ Fettsäuren. Sie bestehen größtenteils aus Triglyceriden mit einem Anteil von höchstens 15 % an Diglyceriden und nicht mehr als 1 % an Monoglyceriden. Bei der Herstellung der erfindungsgemäßen Hartfette werden zunächst pflanzliche Fette nach Reinigung in Fettsäuren und Glycerin mit Hilfe von Wasser bei hoher Temperatur zerlegt. Die Fettsäurenmischung wird hydriert, fraktioniert und Vakuum-destilliert, vor allem um kurzkettige Fettsäuren zu entfernen. Die bevorzugt verwendeten C₁₂-C₁₈ Fettsäuren werden auf eine geeignete Mischung eingestellt und mit gereinigtem Glycerin verestert. Diese Reaktionsmischung wird anschließend weiter gereinigt, insbesondere durch Waschen, Vakuum-Trocknen, Behandlung zur Entfernung von Farbstoffen und Dampfdestillation. Bei den bevorzugt verwendeten Hartwachsen beträgt der Anteil der C₁₂-C₁₈ Fettsäureketten wenigstens 85 %, bevorzugt wenigstens 90 %. Wichtig ist auch, dass der Hydroxylwert der Hartfette bevorzugt weniger als etwa 10 beträgt. Für den Hydroxylwert verantwortlich sind hauptsächlich die Monoglyceride, da diese zwei Hydroxylgruppen vom Glycerinrest zur Verfügung stellen und Diglyceride, die über eine freie Hydroxylgruppe verfügen. Gemessen werden kann der Hydroxylwert durch die Bestimmung der Menge an KOH, die benötigt wird, um die Menge an Essigsäure zu neutralisieren, die durch die Acetylierung des Hartfettes verbraucht wird. Der Hydroxylwert gibt also die Anzahl der freien Hydroxylgruppen in der Hartfettgrundlage an. Da die erfindungsgemäß eingesetzten Hartfette üblicherweise während der Reinigung von Glycerin befreit werden, ist der Hydroxylwert ein Indikator für die Gegenwart von Mono- und/oder Diglyceriden, die in der Hartfettmischung vorhanden sind. Sofern weitere Zusatzstoffe in der Hartfettmischung vorhanden sind, die freie Hydroxylgruppen beisteuern, beeinflussen diese ebenfalls den Hydroxylwert.

Ein weiterer wichtiger Kennwert der erfindungsgemäß verwendeten Hartfette ist der Jodwert. Der Jodwert gibt die Anzahl der Gramm an Halogen (Jod) wieder, das von 100 g der Hartfettmischung verbraucht wird. Für den Verbrauch des Halogens kommen in erster Linie die ungesättigten Verbindungen, also ungesättigte Fettsäuren in Frage. Da erfindungsgemäß dieser Anteil sehr gering ist, liegt bei den einzusetzenden Qualitäten die Jodzahl unter 3, bevorzugt unter 2.

Ein wesentlicher Parameter der Suppositoriengrundlage ist auch der Peroxidwert. Dieser Wert spiegelt die Menge an Peroxid in Milliäquivalenten von aktivem Sauerstoff wieder, die in 1000 g der Suppositoriengrundlage vorhanden sind. Bei den erfindungsgemäß verwendeten Suppositoriengrundlagen beträgt der Peroxidwert, angegeben in meq O/kg maximal 5, bevorzugt maximal 3 und besonders bevorzugt maximal 1.

Neben den lipidhaltigen Suppositoriengrundlagen werden auch wasserlösliche Massen auf Macrogolbasis eingesetzt, die sich in den rektal vorhandenen Flüssigkeiten lösen. Bevorzugt werden Macrogol 6000 bzw. Mischungen hoher und niedriger Molekülmassen eingesetzt. Die Anteile der Macrogol basierten Zusatzstoffe liegen zwischen 0 und 20, bevorzugt zwischen 0 und 5 und besonders bevorzugt bei 0,1 bis 3 Gew.-% bezogen auf die fertige Formulierung. Wenn derartige Komponenten eingesetzt werden sollten, muss auf einen niedrigen Peroxidwert von maximal 5 meq O/kg geachtet werden.

Budesonid ist ein Glucocorticoid mit hoher lokaler antientzündlicher Wirksamkeit. Die Substanz ist praktisch unlöslich in Wasser (0,014 mg/ml, Merck Index), auf Grund seiner lipophilen Eigenschaften lösen sich jedoch nennenswerte Mengen in organischen Lösungsmitteln wie Ethanol, Methanol und Chloroform. In Abhängigkeit vom verwendeten Medium ist die gelöste Substanz mehr oder weniger instabil. Diese Instabilität ist unter anderem eine Folge eines oxidativen Abbaus von Budesonid. Ohne weitere Maßnahme ist daher die allgemein bekannte Anwendung von lipidhaltigen oder wasserlöslichen Grundlagen keine Option für die Herstellung von stabilen und verträglichen Budesonid Suppositorien, da der in diesen Vehikeln gelöste arzneilich wirksame Bestandteil rasch abgebaut wird.

Wie in Beispiel 1 beschrieben, zeigen einfache Budesonid Suppositorien aus Hartfett unterschiedlicher Qualität (Witepsol® H15, Witepsol® W45) bereits nach einer Lagerung von 3 Monaten bei 25°C/60% relativer Feuchte eine Gehaltsabnahme von ca. 10%. Mischungen von Budesonid mit Macrogolen sind auf Grund der in dieser Matrix inhärent in Spuren vorhandenen bzw. gebildeten Peroxide per se unverträglich und stellen wegen der lokalen Schleimhautreizung, die diese Suppositorien nach der Anwendung auslösen, keine Alternative für die Behandlung von entzündlichen Erkrankungen des Rektums dar.

Die erfindungsgemäßen Budesonid Suppositorien dagegen zeigen die dem Stand der Technik zu Grunde liegenden Nachteile nicht. Dabei wird die vorliegende Erfindung nur durch eine Kombination von Maßnahmen, also wenigstens zwei, bevorzugt wenigstens drei der unten aufgeführten Maßnahmen ermöglicht, von denen jede einzelne alleine nicht ausreicht, das vorgegebene Ziel zu erreichen.

Gegenstand der vorliegenden Erfindung sind daher stabile und verträgliche Budesonid Suppositorien, die erhalten werden durch wenigstens zwei der folgenden Maßnahmen:
(a) die Verwendung von Budesonid als arzneilich wirksamer Bestandteil in geeigneter Partikelgrößenverteilung,
(b) die Auswahl einer geeigneten Hartfettqualität,
(c) den Zusatz von Ascorbylpalmitat als Antioxidanz zur Hartfettgrundlage in einer optimierten Konzentration,
(d) die Einstellung eines optimalen Verhältnisses von in der Hartfettgrundlage gelösten sowie suspendierten Budesonids,
(e) die Verwendung einer für die beschriebene Anwendung optimalen Suppositorienform bzw. -größe und
(f) die Verwendung einer Gießfolie mit geringerer Permeabilität für Sauerstoff als Packmittel.

Die gemäß der erfindungsgemäßen Anmeldung zusammengesetzten und hergestellten Budesonid Suppositorien haben eine ausreichende Stabilität, die die Lagerung und Anwendung der Suppositorien bei Umgebungsbedingungen von 25°C/60% relativer Feuchte über mindestens 24 Monate ermöglichen. Gleichzeitig wird durch die Erfindung sichergestellt, dass die Anwendung der Suppositorien für die Behandlung der erfindungsgemäßen Indikation auf Grund ihrer Größe und Form schmerzfrei erfolgen kann und, dass nach der Einführung der Suppositorien der Anteil an suspendiertem arzneilich wirksamen Bestandteil rasch aus der geschmolzenen Grundlage auf die betroffenen Schleimhautstellen sedimentiert während der gelöste Anteil sich aus der Grundlage heraus verteilt. Damit ist sichergestellt, dass sowohl die Patienten-Compliance als auch die Wirksamkeit von Budesonid über einen ausreichend langen Zeitraum ermöglicht wird.

Für die Herstellung von stabilen Budesonid Suppositorien eignen sich besonders lipophile Grundlagen des Hartfett-Typs. Als ganz besonders bevorzugt haben sich Hartfett-Typen mit hohem Anteil an Triglyceriden (wenigstens ca. 85 %) und damit niedriger Hydroxylzahl (5-15) herausgestellt. Hartfette bestehen grundsätzlich aus einem Gemisch von verschiedenen Mono-, Di- und Triglyceriden. Je nach Zusammensetzung des Hartfetts ergibt sich daraus eine unterschiedliche Anzahl freier OH-Gruppen, woraus unterschiedliche Eigenschaften resultieren. Eine Kennzahl für Hartfette ist daher die Hydroxylzahl. Die größte Bedeutung haben Hartfetttypen mit Hydroxylzahlen < 15. Wegen der geringen Anzahl an freien OH-Gruppen kommt es bei diesen Suppositorien-Grundlagen kaum zu Inkompatibilitäten mit Hydrolyse-empfindlichen Stoffen oder Stoffen mit freien Säuregruppen. Im Allgemeinen weisen dagegen Hartfette mit hoher Hydroxylzahl eine gute Emulgierfähigkeit und kaum Rissbildung beim Erstarren auf. Eine Neigung zum Nachhärten ist allerdings zu beobachten und Inkompatibilitäten mit den Wirkstoffen sind möglich. Hartfette mit einer niedrigen Hydroxylzahl von < 15 haben jedoch eine geringere Emulgierfähigkeit, neigen häufiger zu Rissbildung beim Erstarren und zeigen wenig Neigung zum Nachhärten.

Erfindungsgemäß werden bevorzugt Hartfetttypen eingesetzt, die möglichst wenig oder keine ungesättigten Fettsäuren beinhalten, weil ungesättigte Verbindungen häufig Oxidationsreaktionen unterliegen und ranzig werden können. Das Maß an ungesättigten Bindungen in einem Lipid kann durch die Jodzahl (Ph.Eur. 2.5.4) ermittelt werden.

Hartfett besteht aus einer Mischung von Mono-, Di- und Triglyceriden. Durch Variationen in der Zusammensetzung und den veresterten Fettsäuren kann der Schmelzpunkt des Hartfettes verändert werden.

Ein bevorzugt eingesetzter handelsüblicher Typ ist Witepsol® H 15. Diese besonders bevorzugte Qualität enthält ganz überwiegend gesättigte Fettsäuren (lodzahl ≤ 3), zeichnet sich durch einen kleinen Abstand zwischen Schmelz- (33,5 - 35,5°C) und Erstarrungspunkt (32,5 - 34,5°C) aus und weist nach dem Ausgießen nur eine geringe Tendenz zum Nachhärten auf. Mit der Verwendung dieser Grundlage wird sichergestellt, dass die Budesonid Suppositorien bei Körpertemperatur schmelzen und den Wirkstoff freisetzen.

Es hat sich überraschenderweise gezeigt, dass erst eine Kombination von in der besonders bevorzugten Hartfettqualität molekular dispers gelösten sowie suspendierten Wirkstoffs die Herstellung von lagerstabilen Budesonid-Zäpfchen ermöglicht. Diese optimierte Kombination aus Lösungs- und Suspensions-Zubereitung in einem Suppositorium erlaubt es, dass weitere Maßnahmen zur Stabilisierung sich lediglich auf den gelösten Anteil des Wirkstoffs beschränken müssen. Wie in Beispiel 2 beschrieben, beträgt die Löslichkeit von Budesonid in der besonders bevorzugten Hartfettqualität Witepsol® H 15 1,5 mg/g. Diese Sättigungskonzentration ermöglicht die Berechnung der Dosislöslichkeit des Wirkstoffes in der Grundlage und damit eine gezielte Auswahl der zur Stabilisierung erforderlichen Menge an Antioxidanz.

Bei einer Budesonid-Dosis von 2 mg bzw. 4 mg und einer Suppositorienmasse von 1,8 g liegen für die 2 mg Form 100 % der Dosis molekular dispers gelöst vor (reines Lösungszäpfchen) während für die 4 mg Form der gelöste Anteil 67,5 % (kombinierte Variante aus Lösungs- und Suspensionszäpfchen) beträgt.

Erst die erfindungsgemäß bevorzugte gezielte Absenkung der Suppositorienmasse von 1,8 g auf 1 g ermöglicht es, für den gewünschten Dosisbereich von 2 mg bis 4 mg die kombinierte Suppositorienvariante zu realisieren. Im Falle der Budesonid 2 mg Suppositorien liegen dann 75% der Dosis in gelöster sowie 25 % in suspendierter Form vor. Für die Budesonid 4 mg Suppositorien liegt das Verhältnis bei 37,5 % (gelöster Anteil) sowie 62,5 % (suspendierter Anteil). Dadurch kann gewährleistet werden, dass eine optimale Konzentration an Antioxidanz zugesetzt werden kann, die die stabilisierende Wirkung ausschließlich auf den gelösten Wirkstoffanteil von 37,5 % bis 75,0 % entfaltet. Erst diese überraschenderweise herausgefundene komplexe Wechselwirkung von physikalischer und chemischer Stabilisierung der Zubereitung ermöglicht die Langzeitstabilität von Budesonid Suppositorien bei Umgebungsbedingungen und damit den Verzicht auf Kühlschranklagerung.

Mit der Reduktion der Masse von 1,8 g auf etwa 1 g erhält das Budesonid Suppositorium gleichzeitig eine für die Anwendung besonders bevorzugte Größe und Form, so dass ein schmerzfreies Einführen gewährleistet werden kann.

Die erfindungsgemäßen Suppositorien weisen ein Gewicht von etwa 0,8 bis 1,2 g, bevorzugt 0,9 bis 1,1 g und besonders bevorzugt von 0,95 bis 1,05 g auf.

Wie in Beispiel 1 beschrieben, muss der in der Hartfettgrundlage molekular dispers gelöste Wirkstoffanteil durch den Zusatz eines antioxidativ wirkenden Hilfsstoffes stabilisiert werden. Antioxidantien sind eine Gruppe von Hilfsstoffen, die als Radikalfänger bzw. als selbst leicht oxidierbare Stoffe fungieren und damit den Wirkstoff vor Oxidation schützen können.

Überraschenderweise wurde nun herausgefunden, dass sich von den in nicht wässrigen, lipophilen Systemen üblicherweise eingesetzten Antioxidanzien wie Ascorbylpalmitat, DL-α-Tocopherol und Butylhydroxyanisol nur Ascorbylpalmitat als geeignet für die Stabilisierung erweist. Beispiel 3 zeigt die Ergebnisse der Auswahlversuche. Auffallend ist, dass die Hilfsstoffe DL-α-Tocopherol und Butylhydroxyanisol im Gegensatz zu dem eigentlich erwünschten antioxidativen Effekt den Abbau von Budesonid in der Hartfettgrundlage sogar noch verstärken.

Als besonders geeignet hat sich die Verwendung von Ascorbylpalmitat in einem Konzentrationsbereich von 50 ppm bis 250 ppm gezeigt. Beispiel 4 zeigt den konzentrationsabhängigen Effekt von Ascorbylpalmitat auf das Verunreinigungsprofil von Budesonid 2 mg Suppositorien während einer Lagerungsdauer von 24 Monaten bei 25°C/60 % relativer Feuchte. Im Vergleich zu den nicht stabilisierten Budesonid Suppositorien aus Beispiel 1 ermöglicht erst diese Maßnahme eine Langzeitstabilisierung des im Hartfett gelösten Wirkstoffanteils, wobei sich bevorzugt ein Konzentrationsbereich von 100 ppm bis 200 ppm Ascorbylpalmitat als wirksam erweist. Mit der beschriebenen Absenkung der Suppositorienmasse auf 1 g sowie dem optimierten Zusatz von Ascorbylpalmitat von 100 ppm lassen sich damit Budesonid Suppositorien herstellen, die mindestens 24 Monate bei 25°C/60 % relativer Feuchte stabil sind und keiner Kühlschranklagerung bedürfen.

In Beispiel 5 ist die Zusammensetzung der bevorzugten Ausführungsformen von Budesonid 2 mg und 4 mg Suppositorien beschrieben. Dabei erfolgt das Ausgießen der aufgeschmolzenen Suppositorienmasse in Gießformen aus Kunststoff, in denen die Masse anschließend rasch erstarrt. Die Dosierung erfolgt für jedes einzelne Suppositorium volumetrisch.

Die zur Aufnahme der Schmelze verwendeten Verbundfolien bestehen bevorzugt aus mit Polyvinylidenchlorid (40 g/m²) und Polyethylen geringer Dichte (40 µm) beschichteten 100 µm-starken Polyvinylchlorid-Folien (LDPE/PVC/PVdC). Diese Gießform ist ein Packmittel mit verstärkter Barrierefunktion gegen Sauerstoff und stellt einen zusätzlichen Schutzmechanismus für die Formulierung dar. Der Barriereschutz kann noch verstärkt werden, wenn Gießformen aus Aluminiumfolie für die Budesonid-Suppositorien gewählt werden.

In einer bevorzugten Ausführungsform werden die erfindungsgemäßen Suppositorien vollständig unter Stickstoffatmosphäre hergestellt. Dies bedeutet, dass nach Zusammenfügen der einzelnen Komponenten der fertigen pharmazeutischen Formulierung Luft evakuiert und anschließend mit Stickstoff oder mit Edelgas begast wird, damit keine oxidativen Reaktionen stattfinden können. Die aufgeschmolzene Suppositorienmasse mit Wirkstoff wird dann direkt in die vorbereiteten gasdichten Verbundfolien verbracht, wo die Aushärtung erfolgt. Die Verbundfolien sind dabei so ausgestaltet, dass sie die fertige Suppositorienform vorgeben und nach Befüllung so verschlossen werden können, dass Sauerstoffkontakt mit der Suppositorienmasse weitestgehend vermieden wird.

Durch die erfindungsgemäßen Maßnahmen ist die Herstellung von bei Raumtemperatur lagerstabilen Budesonid-Suppositorien möglich. Die Ergebnisse der Haltbarkeitsuntersuchungen von Beispiel 6 belegen beeindruckend, dass die gewählte Kombination von Stabilisierungsmaßnahmen und Schutzmechanismen die Bereitstellung von stabilen Budesonid Suppositorien ermöglicht.

Die Partikelgröße des Wirkstoffes Budesonid sollte möglichst klein sein. Dazu wird das Budesonid in einer geeigneten Mühle (z.B. Strahlmühle) mikronisiert. Erfindungsgemäß wird die Mikronisierung so durchgeführt, dass 100 % der Teilchen kleiner als 10 µm sind. Das mikronisierte Budesonid wird über die Pulvereintragsstation eines Inline-Homogenisators in das geschmolzene Hartfett eingearbeitet. Dabei werden Partikelaggregate zerkleinert und es kommt zu einer gleichmäßigen Verteilung des ungelösten Anteils des Wirkstoffes in der Grundlage. Die Reduktion der Partikelgröße ist auch ein geeignetes Mittel, eine Wirkstoffsedimentation im Ansatz zu verhindern. Wichtig ist auch, dass sich die Partikel nicht zusammenlagern. Die Gefahr der Partikelagglomeration ist im Allgemeinen umso größer, je kleiner die Partikel sind, da mit abnehmender Partikelgröße die Oberfläche der einzelnen Partikel zunimmt und damit die Oberflächenenergie steigt. Durch die Zugabe einer kleinen Menge eines Tensids (üblicherweise weniger als 0,5 Gew.-% bezogen auf die mikronisierte Budesonidpräparation) kann die Gefahr des Partikelwachstums verhindert werden. Die Zugabe eines Tensids kann auch zu einer besseren Spreitung und Benetzung des Wirkstoffes in der Rektalflüssigkeit führen. Wichtig ist allerdings, dass ein Tensid gewählt wird, das keine unerwünschte Nebenreaktion bei der Verabreichung des Suppositoriums verursacht.

Für die therapeutische Anwendung wird der arzneilich wirksame Bestandteil Budesonid in Dosierungen von 2 mg bis 4 mg eingesetzt. Der Wirkstoff wird dabei in mikronisierter Form verwendet, wobei 100% der Teilchen kleiner als 10 µm, mindestens 95% kleiner als 5 µm und mindestens 80% kleiner als 3 µm sind. Die Bestimmung der Partikelgrößenverteilung von Budesonid erfolgt durch Laserbeugungsanalyse (Laserdiffraktometrie). Dabei wird Budesonid in einem wässrigen Medium nassdispergiert. Nach Bestrahlung der Partikel mit monochromatischem Laserlicht wird das Beugungsmuster bestimmt, aus dem anschließend die Partikelgrößenverteilung berechnet werden kann. Die Verwendung von mikronisierter Qualität verhindert die Sedimentation von suspendiertem Budesonid in der aufgeschmolzenen Suppositorienmasse während der Herstellung und ermöglicht damit die gleichmäßige Wirkstoffverteilung in den ausgegossenen und erstarrten Formen. Gleichzeitig wird durch die Mikronisierung die Auflösungsgeschwindigkeit des nach Einführen und Schmelzen des Suppositoriums auf die Schleimhaut sedimentierten Budesonids in der Rektalflüssigkeit erhöht. Beispiel 7 zeigt die Ergebnisse von *in* vitro-Freisetzungsversuchen von Budesonid 2 mg Suppositorien über einen Zeitraum von 2 Stunden. Innerhalb dieses Zeitraums wird sowohl der suspendierte also auch der in der Hartfettgrundlage gelöste Anteil des Wirkstoffs freigesetzt. Mit der erfindungsgemäßen Formulierung wird somit sichergestellt, dass die gesamte Budesonid-Dosis aus der Formulierung freigesetzt wird und über einen ausreichend langen Zeitraum auf der Rektalschleimhaut verfügbar ist und somit eine therapeutische Wirkung erreicht wird.

Die erfindungsgemäßen Suppositorien werden bevorzugt zur Behandlung von entzündlichen Erkrankungen des Rektums verwendet. Hierbei handelt es sich in bevorzugter Weise um akute Erkrankungen, bei denen eine schnelle Linderung der Beschwerden wünschenswert ist.

Die erfindungsgemäßen Suppositorien werden vorzugsweise zur Behandlung von Patienten mit aktiver ulzerativer Proctitis eingesetzt. In bevorzugter Ausführungsform wird ein Suppositorium mit 2 mg Budesonid oder ein Suppositorium mit 4 mg Budesonid einmal am Morgen und einmal am Abend verabreicht. Die erfindungsgemäßen Budesonid-Suppositorien sind daher für die Behandlung von aktiver ulzerativer Proctitis besonders geeignet.

In einer klinischen Studie bei Patienten mit akuter, ulzerativer Proctitis, in der Ausführungsformen der vorliegenden Erfindung entweder alleine oder als Kombinationstherapie mit konventionellen Mesalazinsuppositorien im Vergleich zu konventionellen Mesalazinsuppositorien untersucht wurden, konnte gezeigt werden, dass die Verwendung der erfindungsgemässen Budesonidsuppositorien zu einer deutlichen Verringerung der Zeit bis zum Abklingen von klinischen Symptomen führte. Dieser Endpunkt wurde in der klinischen Studie definiert als der erste Tag von drei aufeinanderfolgenden Tagen mit eine Punktescore von 0 für rektale Blutung und der Stuhlfrequenz.

Die Rate der Patienten, die eine klinische und endoskopische Remission oder eine Verbesserung Ihre Symptome zeigten, war in der mit Budesonid-Suppositorien behandelten höher. Klinische und endoskopische Remission war dann erreicht, wenn die Patienten im modifizierten DAI-UC Index (Disease Activity Index-Ulcerative Colitis) einen Wert von ≤ 1 hatten, wobei das Ergebnis in den Kategorien rektale Blutung und Stuhlfrequenz 0 sein musste und mindestens 1 Punkt Reduktion in der Unterkategorie "Erscheinungsbild der Mukosa" erreicht werden musste. Bei einer Verbesserung der Symptome war mindestens eine Verringerung des Gesamtscores von ≥ 3 Punkten gefordert.

Die Handhabung der erfindungsgemäßen Budesonid Suppositorien mit reduzierter Suppositorienmasse erreichte bei den Patienten auch eine höhere Akzeptanz verglichen mit den Mesalazin Suppositorien, die als Vergleichspräparat getestet wurden.

In einer besonderen Ausführungsform der vorliegenden Erfindung werden die erfindungsgemäßen Budesonid-Suppositorien im Rahmen einer Kombinationstherapie mit Mesalazin-Suppositorien verwendet. Eine derartige Kombinationstheraphie besteht bevorzugt darin, dass jeweils am Morgen ein Budesonid-Zäpfchen und am Abend ein Mesalazin-Zäpfchen verabreicht wird oder, dass am Morgen ein Mesalazin-Zäpfchen und am Abend ein Budesonid-Zäpfchen verabreicht wird.

Für die Beurteilung der Wirksamkeit der Suppositorien wird der modifizierte UC-DAI Bewertungsstandard, wie von Kamm et al. (2007, Gastroenterology, 132, S. 66-75) verwendet. Auf die dort offenbarte Tabelle 1 und die zugehörigen Definitionen der Parameter wird ausdrücklich verwiesen.

Bevorzugte Ausführungsformen der vorliegenden Erfindung werden durch die nachfolgenden Beispiele verdeutlicht.

### Beispiel 1: Einfluss des Hartfettes auf den Abbau von Budesonid in Hartfett unterschiedlicher Qualitäten ohne Stabilisierung

**Tabelle 1**

| **Charge** | **Zusammensetzung** | | **Gehalt [%]** | | |
|---|---|---|---|---|---|
| | | | Initial | 3 Monate 25°C/60% rel. Feuchte | 3 Monate 30°C/65% rel. Feuchte |
| | | | | | |
| V1860 | Budesonid | 2 mg | | | |
| | Witepsol® H15 | 1798 mg | 96,2 | 86,6 | 75,3 |
| | Gesamt: | 1800 mg | | | |
| | | | | | |
| V1866 | Budesonid | 2 mg | | | |
| | Witepsol® W45 | 1798 mg | 94,4 | 77,5 | 70,2 |
| | Gesamt: | 1800 mg | | | |

Ohne weitere Stabilisierungsmaßnahmen sind Budesonid 2 mg Suppositorien vom Hartfett-Typ instabil, wobei auch die Natur des eingesetzten Hartfettes einen Einfluss auf die Stabilität hat. Bereits nach einer Lagerungsdauer von 3 Monaten zeigt sich bei 25°C/60 % relativer Feuchte eine Gehaltsabnahme von 10 % und mehr. Die Stabilität von Budesonid mit dem Hartfett-Typ Witepsol® W45 ist dabei deutlich ungünstiger als mit der Qualität Witepsol® H15. Witepsol® H15 weist auf Grund des geringen Anteils an Mono- und Diglyceriden eine niedrige Hydroxylzahl auf, während Witepsol® W45 einen höheren Anteil an Mono- und Diglyceriden hat. Damit reduziert sich für Witepsol® H15 die Möglichkeit einer Interaktion zwischen den freien Hydoxylgruppen des Hartfetts und den funktionellen Gruppen des Wirkstoffmoleküls.

### Beispiel 2: Sättigungslöslichkeit von Budesonid im Hartfett-Typ Witepsol® H15

Die Löslichkeit von Budesonid wurde in geschmolzenem Witepsol® H15 bei 40°C bestimmt. Dabei wurden steigende Mengen an Budesonid in der Hartfettgrundlage suspendiert. Nach Abtrennung des ungelösten Anteils und Erstarren der Suppositorienmasse wurde der gelöste bzw. ungelöste Anteil mit HPLC/UV wie folgt bestimmt:

**Tabelle 2**

| **Stärke der Suppositorien/Dosierung** | **In Witepsol® H15 gelöster Anteil an Budesonid** |
|---|---|
| 1 mg Budesonid in 1 g Witepsol® H15 | 0,97 mg/g entsprechend 100% |
| 2 mg Budesonid in 1 g Witepsol® H15 | 1,47 mg/g entsprechend 75% |
| 3 mg Budesonid in 1 g Witepsol® H15 | 1,64 mg/g entsprechend 55% |
| 4 mg Budesonid in 1 g Witepsol® H15 | 1,65 mg/g entsprechend 42% |

Die Sättigungskonzentration von Budesonid in Witepsol® H15 bestimmt bei 40°C beträgt demnach ca. 1,5 mg/g. In 1 g-Suppositorium mit einer Dosierung von 2 mg Budesonid sind 75 % des Wirkstoffs in der Hartfettgrundlage molekular dispers gelöst, bei einer Dosierung von 4 mg sind es 55 %. Somit liegt bei den erfindungsgemäßen Budesonid Suppositorien eine Mischung aus Lösungs- und Suspensions-Zubereitung vor. Nur der gelöste Anteil an Budesonid muss durch den Zusatz von Antioxidantien stabilisiert werden.

Für die Bestimmung der Sättigungskonzentration von Budesonid in Witepsol® H15 wurden 1 g-Suppositorium mit unterschiedlichen Budesonid-Dosierungen hergestellt und über eine Dauer von 24 Stunden bei 40°C stehen gelassen. Anschließend erfolgte eine Zentrifugation (10 Minuten bei 4000 UpM), um ungelöstes Budesonid zu sedimentieren. Zuletzt wurden die Suppositorien über 2 Stunden im Kühlschrank (2-8°C) ausgehärtet. Zur Bestimmung der Budesonid-Konzentration wurden die Suppositorien in unterschiedlichen Verhältnissen zweigeteilt (unterer Teil mit Suppositorienspitze und oberer Teil). Die beiden Teile wurden einzeln für die HPLC-Gehaltsbestimmung aufgearbeitet und dann analysiert. Aus den einzelnen Einwaagen sowie den erhaltenen Gehaltswerten wurde die Budesonid-Konzentration in mg/g berechnet. Der gelöste Anteil an Budesonid verteilt sich homogen in der Suppositoriengrundlage, während sich der ungelöste Anteil in der Suppositorienspitze anreichert. Somit geben die Ergebnisse der Budesonid-Gehaltsbestimmung im oberen Teil der Suppositorien den gelösten Anteil von Budesonid wieder. Diese Werte finden sich in der Tabelle.

### Beispiel 3: Auswahl eines geeigneten Antioxidanz zur Stabilisierung von gelöstem Budesonid im Hartfett-Typ Witepsol® H15

Für die Auswahlversuche wurden unterschiedliche, im Stand der Technik beschriebene Antioxidanzien getestet. Dabei wurden Budesonid 2 mg Suppositorien mit einer Masse von 1,8 g verwendet, die den Wirkstoff vollständig in der Hartfettgrundlage gelöst enthalten. Die Antioxidantien wurden in einer Konzentration von 100 ppm der Zubereitung zugesetzt. Der Kontrollansatz war frei von Antioxidanz. Als Gießformen wurden Verbundfolien bestehend aus mit Polyethylen geringer Dichte (LDPE) beschichteten Polyvinylchlorid-Folien (PVC) verwendet, die keine zusätzliche Barrierschicht aus Polyvinylidenchlorid (PVdC) enthielten. Die Suppositorien wurden bei 30°C/65 % relativer Feuchte über eine Dauer von 30 Tagen gelagert. Nach der Herstellung und Lagerung wurde das Verunreinigungsprofil der Budesonid Suppositorien mit HPLC/UV bestimmt. Die beiden folgenden Tabellen fassen die getesteten Formulierungen sowie die Ergebnisse zusammen.

**Tabelle 3**

| **Charge:** | **V1912** | **V1915** | **V1916** | **V1917** |
|---|---|---|---|---|
| **Zusammensetzung:** | | | | |
| Budesonid, mikronisiert¹ | 2 mg | 2 mg | 2 mg | 2 mg |
| Ascorbylpalmitat | 0,180 mg (100 ppm) | --- | --- | --- |
| DL-α-Tocopherol | --- | 0,180 mg (100 ppm) | --- | --- |
| Butylhydroxyanisol | --- | --- | 0,180 mg (100 ppm) | --- |
| Witepsol® H15 | 1797,820 mg | 1797,820 mg | 1797,820 mg | 1798,000 mg |
| Suppositorienmasse | 1800,000 mg | 1800,000 mg | 1800,000 mg | 1800,000 mg |

| | | | | |
|---|---|---|---|---|
| ¹ Partikelgrößenverteilung: 100 % < 10 µm, ≥ 95 % < 5 µm, ≥ 80 % < 3 µm | | | | |

**Tabelle 4**

| **Charge:** | **V1917 (Kontrolle)** | | **V1912 (100 ppm Ascorbylpalmitat)** | | **V1915 (100 ppm DL-α-Tocopherol** | | **V1916 (100 ppm Butylhydroxyanisol** | |
|---|---|---|---|---|---|---|---|---|
| **Lagerdauer bei 30°C/65% rel. Feuchte in Tagen** | 0 | 30 | 0 | 30 | 0 | 30 | 0 | 30 |
| **Summe an Abbauprodukten (%)** | 1,34 | 6,03 | 0,73 | 0,65 | 2,13 | 14,10 | 1,89 | 15,52 |
| **Zunahme während der Lagerung (%)** | 4,69 | | --- | | 11,97 | | 13,63 | |

Ohne Zusatz eines Antioxidanz (Kontrollansatz, Charge V1917) zeigt sich innerhalb von 30 Tagen bei 30°C/65 % relativer Feuchte ein Budesonid-Abbau von ca. 5 % (siehe auch Beispiel 1). Der Zusatz von 100 ppm DL-α-Tocopherol und Butylhydroxyanisol führt zu keiner Stabilisierung der Suppositorien (siehe Chargen V1915 und V1916). Überraschenderweise nimmt der Abbau des Wirkstoffes in Gegenwart dieser Antioxidanzien sogar deutlich zu. Somit stellen DL-α-Tocopherol und Butylhydroxyanisol keine Optionen zur Stabilisierung der Budesonid Suppositorien dar. Ascorbylpalmitat dagegen zeigt einen deutlichen antioxidativen Effekt. Während der Lagerdauer zeigt sich kein Abbau von Budesonid. Die Durchführung des Versuchs erfolgte mit für die Budesonid-Stabilität ungünstigsten Bedingungen, wie vollständige Löslichkeit des Wirkstoffs im Hartfett sowie die Verwendung von Gießfolien ohne weitere Sauerstoffbarriere, um den antioxidativen Effekt von Ascorbypalmitat zeigen zu können.

### Beispiel 4: Optimale Konzentration an Ascorbylpalmitat als Antioxidanz zur Stabilisierung von gelöstem Budesonid im Hartfett-Typ Witepsol® H15

Der Effekt von Ascorbylpalmitat als Antioxidanz zur Stabilisierung von Budesonid wurde mit folgenden Rezepturen von Budesonid 2 mg Suppositorien getestet:

**Tabelle 5**

| **Charge:** | **V2035** | **V2034** | **V2036** |
|---|---|---|---|
| **Zusammensetzung:** | | | |
| Budesonid, mikronisiert¹ | 2 mg | 2 mg | 2 mg |
| Ascorbylpalmitat | 0,075 mg (75 ppm) | 0,100 mg (100 ppm) | 0,125 mg (125 ppm) |
| Witepsol® H15 | 997,925 mg | 997,900 mg | 997,875 mg |
| Suppositorien masse | 1000,000 mg | 1000,000 mg | 1000,000 mg |

| | | | |
|---|---|---|---|
| ¹ Partikelgrößenverteilung: 100 % < 10 µm, ≥ 95 % < 5 µm, ≥ 80 % < 3 µm | | | |

Für alle Suppositorien beträgt damit der Anteil an in der Hartfettgrundlage gelöstem und zu stabilisierendem Budesonid 75 %. Als Gießformen wurden Verbundfolien aus LDPE/PVC/PVdC verwendet. Nach der Herstellung sowie einer Lagerung von 24 Monaten bei 25°C/60 % relativer Feuchte wurde das Verunreinigungsprofil der Budesonid Suppositorien mit HPLC/UV bestimmt. Dabei wurden folgende Ergebnisse erhalten:

**Tabelle 6**

| **Charge:** | **V2035 (75 ppm Ascorbyl palmitat)** | | **V2034 (100 ppm Ascor bylpalmitat)** | | **V2036 (125 ppm Ascor bylpalmitat)** | |
|---|---|---|---|---|---|---|
| **Lagerdauer in Monaten** | 0 | 24 | 0 | 24 | 0 | 24 |
| **Summe an Abbauprodukten (%)** | 0,19 | 2,93 | 0,16 | 2,17 | 0,19 | 1,70 |
| **Zunahme während der Lagerung (%)** | 2,74 | | 2,01 | | 1,51 | |

Ascorbylpalmitat stabilisiert das in der Hartfettgrundlage molekular dispers gelöste Budesonid konzentrationsabhängig. Die bevorzugte Ausführungsform von Budesonid 2 mg und 4 mg Suppositorien enthält Ascorbylpalmitat in einem Konzentrationsbereich von 100 ppm bis 150 ppm.

### Beispiel 5: Qualitative und quantitative Zusammensetzung der bevorzugten Ausführungsformen von Budesonid 2 mg und 4 mg Suppositorien

**Tabelle 7**

| **Zusammensetzung** | | |
|---|---|---|
| Budesonid, mikronisiert¹ | 2 mg | 4 mg |
| Ascorbylpalmitat | 0,10 - 0,15 mg | 0,10 - 0,15 mg |
| | (100 - 150 ppm) | (100 - 150 ppm) |
| Witepsol® H15 | 997,85 - 997,900 mg | 995,85 - 995,90 mg |
| Suppositorienmasse | 1000,00 mg | 1000,00 mg |
| Gießform | Verbundfolie aus-LDPE/PVC/PVdC | Verbundfolie aus LDPE/PVC/PVdC |

| | | |
|---|---|---|
| ¹ Partikelgrößenverteilung: 100 % < 10 µm, ≥ 95 % < 5 µm, ≥ 80 % < 3 µm | | |

### Beispiel 6: Haltbarkeitsversuche der bevorzugten Ausführungsformen von Budesonid 2 mg und 4 mg Suppositorien

Budesonid 2 mg und 4 mg Suppositorien wurden in der bevorzugten Ausführungsform mit 100 ppm Ascorbylpalmitat hergestellt und bei 25°C/60 % relativer Feuchte für Haltbarkeitsversuche eingelagert. Nach der Herstellung sowie in regelmäßigen Intervallen während der Lagerung wurden der Gehalt sowie die Reinheit der Suppositorien mit HPLC/UV bestimmt. Die beiden folgenden Tabellen fassen die Ergebnisse für Budesonid 2 mg Suppositorien und Budesonid 4 mg Suppositorien zusammen.

**Tabelle 8**

| **Budesonid 2 mg Suppositorien mit 100 ppm Ascorbylpalmitat, Charge V2042** | | | | | | | |
|---|---|---|---|---|---|---|---|
| | **Lagerzeit (Monate) bei 25°C/60 % rel. Feuchte** | | | | | | |
| | 0 | 3 | 6 | 9 | 12 | 18 | 24 |
| **Gehalt Budesonid (%)** | 99,5 | 97,3 | 96,2 | 96,5 | 96,0 | 95,0 | 96,6 |
| **Summe an Abbauprodukten (%)** | 0,16 | 0,09 | 0,16 | 0,27 | 0,36 | 0,72 | 0,86 |

**Tabelle 9**

| **Budesonid 4 mg Suppositorien mit 100 ppm Ascorbylpalmitat, Charge V2043** | | | | | | | |
|---|---|---|---|---|---|---|---|
| | **Lagerzeit (Monate) bei 25°C/60 % rel. Feuchte** | | | | | | |
| | 0 | 3 | 6 | 9 | 12 | 18 | 24 |
| **Gehalt Budesonid (%)** | 99,4 | 99,0 | 99,6 | 99,6 | 98,0 | 98,0 | 97,6 |
| **Summe an Abbauprodukten (%)** | 0,10 | 0,10 | 0,10 | 0,16 | 0,24 | 0,38 | 0,38 |

Der Budesonid-Gehalt sowie die Summe an Abbauprodukten verändern sich nur unwesentlich während der Lagerung. Mit der überraschenderweise herausgefundenen Kombination von physikalischer und chemischer Stabilisierung ist die Haltbarkeit der Budesonid 2 mg und 4 mg Suppositorien mindestens über eine Dauer von 24 Monaten bei 25°C/60 % relativer Feuchte gewährleistet.

### Beispiel 7: In vitro-Freisetzungsversuche von Budesonid 2 mg Suppositorien

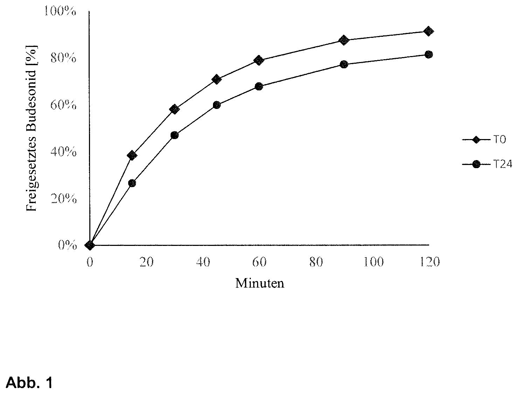

Die erfindungsgemäß zusammengesetzten und hergestellten Budesonid Suppositorien geben den Wirkstoff über einen Zeitraum von 2 Stunden frei. Innerhalb dieses Zeitraums wird sowohl der suspendierte und in mikronisierter Form vorliegende als auch der in der Hartfettgrundlage molekular dispers gelöste Wirkstoffanteil freigesetzt. Damit ist sichergestellt, dass der Wirkstoff über einen ausreichend langen Zeitraum auf der Rektalschleimhaut verfügbar ist und seine therapeutische Wirkung entfalten kann. Die Abbildung 1 zeigt das Freisetzungsprofil von Budesonid 2 mg Suppositorien der Charge V2042 nach der Herstellung (T0) sowie nach einer Lagerung von 24 Monaten bei 25°C/60 % relativer Feuchte (T24). Die Bestimmung erfolgt bei 37°C bevorzugt mit der im Europäischen Arzneibuch beschriebenen Durchflusszelle (Apparatur 4), die mit einer Flussrate von 16 ml/min als geschlossenes System betrieben wird. Als Medium wird Zitronensäure-Phosphatpuffer pH 6,8 mit einem Zusatz von 0,5 % Natriumdodecylsulfat verwendet. Um die Freisetzungskinetik beschreiben zu können, erfolgt die Entnahme der Proben nach 15, 30, 45, 60, 90 und 120 Minuten. Das im Freisetzungsmedium aufgelöste Budesonid wird mit HPLC/UV bestimmt.

## Patentansprüche

1. Pharmazeutische Formulierung zur rektalen Verabreichung, die Budesonid oder ein pharmazeutisch verträgliches Salz oder Derivat davon und wenigstens 80 Gew.-% eines Hartfettes oder eines Gemisches verschiedener Hartfette bezogen auf das Gesamtgewicht der Formulierung sowie wenigstens ein damit verträgliches Antioxidationsmittel enthält, **dadurch gekennzeichnet, dass** das Hartfett einen hohen Anteil von Triglyceriden, nämlich von ≥ 80 Gew.-%, eine Hydroxylzahl von 1 bis 15 aufweist und weniger als 1 Gew.-% ungesättigte Fettsäuren beinhaltet.

2. Formulierung nach Anspruch 1, **dadurch gekennzeichnet, dass** sie einen kleinen Abstand zwischen Schmelz- und Erstarrungspunkt aufweist, wobei der Schmelzpunkt zwischen 33,5°C und 35,5°C und der Erstarrungspunkt zwischen 32,5°C und 34,5°C liegt.

3. Formulierung nach einem der Ansprüche 1 bis 2, **dadurch gekennzeichnet, dass** es sich um ein Suppositorium für die anale Verabreichung handelt.

4. Formulierung nach Anspruch 3, **dadurch gekennzeichnet, dass** sie 1,8 bis 4,2 mg Budesonid pro Suppositorium enthält.

5. Formulierung nach Anspruch 4, **dadurch gekennzeichnet, dass** sie 1,8 bis 2,2 mg Budesonid pro Suppositorium enthält.

6. Formulierung nach Anspruch 4, **dadurch gekennzeichnet, dass** das Gewicht pro Suppositorium zwischen 3,8 und 4,2 mg Budesonid liegt.

7. Formulierung nach einem der vorhergehenden Ansprüche, **dadurch gekennzeichnet, dass** das Gewicht pro Suppositorium zwischen 0,8 und 1,2 g liegt.

8. Formulierung nach einem der Ansprüche 1 bis 7, **dadurch gekennzeichnet, dass** das Antioxidationsmittel Ascorbylpalmitat ist.

9. Formulierung nach Anspruch 7, **dadurch gekennzeichnet, dass** das Ascorbylpalmitat in einer Konzentration von 50 ppm bis 200 ppm vorliegt.

10. Formulierung nach einem der Ansprüche 1 bis 9, **dadurch gekennzeichnet, dass** das Budesonid in mikronisierter Form vorliegt, wobei 100 % der Partikel kleiner als 10 µm pro Partikel sind.

11. Formulierung nach einem der Ansprüche 1 bis 10, **dadurch gekennzeichnet, dass** die lagerstabile pharmazeutische Formulierung unter Ausschluss von Sauerstoff hergestellt wurde.

12. Formulierung nach einem der Ansprüche 1 bis 11, **dadurch gekennzeichnet, dass** die pharmazeutische Formulierung in Suppositorienform in einer gasundurchlässigen Giessfolie verpackt ist.

13. Pharmazeutische Formulierung nach einem der Ansprüche 1 bis 12 zur Verwendung bei der Behandlung von entzündlichen Erkrankungen des Rektums.

14. Pharmazeutische Formulierung nach einem der Ansprüche 1 bis 12 zur Verwendung bei der Behandlung von akuter ulzerativer Proctitis.

15. Pharmazeutische Formulierung zur Verwendung nach einem der Ansprüche 13 oder 14 in Kombination mit Mesalazin-Suppositorien.

## Claims

1. A pharmaceutical formulation for rectal administration, containing budesonide or a pharmaceutically compatible salt or derivative thereof, and at least 80 wt% of a solid fat or a mixture of different solid fats, based on the total weight of the formulation, as well as at least one anti-oxidation agent that is compatible therewith, **characterized in that** the solid fat has a high proportion of triglycerides, namely of ≥ 80 wt%, a hydroxyl value of 1 to 15, and contains less than 1 wt% of unsaturated fatty acids.

2. The formulation according to claim 1, **characterized in that** it has a small distance between melting and freezing point, wherein the melting point is between 33.5°C and 35.5°C and the freezing point is between 32.5°C and 34.5°C.

3. The formulation according to any of claims 1 to 2, **characterized in that** it is a suppository for anal administration.

4. The formulation according to claim 3, **characterized in that** it contains 1.8 to 4.2mg of budesonide per suppository.

5. The formulation according to claim 4, **characterized in that** it contains 1.8 to 2.2mg of budesonide per suppository.

6. The formulation according to claim 4, **characterized in that** the weight per suppository is between 3.8 and 4.2mg of budesonide.

7. The formulation according to any of the preceding claims, **characterized in that** the weight per suppository is between 0.8 and 1.2g.

8. The formulation according to any of claims 1 to 7, **characterized in that** the anti-oxidation agent is ascorbyl palmitate.

9. The formulation according to claim 7, **characterized in that** the ascorbyl palmitate is present in a concentration of 50ppm to 200ppm.

10. The formulation according to any of claims 1 to 9, **characterized in that** the budesonide is present in a micronized form, wherein 100% of the particles are smaller than 10µm per particle.

11. The formulation according to any of claims 1 to 10, **characterized in that** the storage-stable pharmaceutical formulation was prepared under exclusion of oxygen.

12. The formulation according to any of claims 1 to 11, **characterized in that** the pharmaceutical formulation is packaged in the form of suppositories in a gas-tight cast film.

13. A pharmaceutical formulation according to any of claims 1 to 12 for use in the treatment of inflammatory diseases of the rectum.

14. A pharmaceutical formulation according to any of claims 1 to 12 for use in the treatment of acute ulcerative proctitis.

15. The pharmaceutical formulation for use according to any of claims 13 or 14 in combination with mesalazine suppositories.

## Revendications

1. Formulation pharmaceutique pour administration rectale, qui contient du budésonide ou un sel ou un dérivé pharmaceutiquement compatible de celui-ci et au moins 80 % en poids d'une graisse dure ou d'un mélange de diverse graisses dures par rapport au poids total de la formulation ainsi qu'au moins un antioxydant qui lui est compatible, **caractérisée en ce que** la graisse dure présente une fraction élevée de triglycérides, à savoir ≥ 80 % en poids, un indice d'hydroxyle de 1 à 15 et contient moins de 1 % en poids d'acides gras insaturés.

2. Formulation selon la revendication 1, **caractérisée en ce qu'**elle présente un petit écart entre le point de fusion et le point de solidification, dans laquelle le point de fusion se situe entre 33,5 °C et 35,5 °C et le point de solidification entre 32,5 °C et 34,5 °C.

3. Formulation selon l'une quelconque des revendications 1 à 2, **caractérisée en ce qu'**il s'agit d'un suppositoire pour administration anale.

4. Formulation selon la revendication 3, **caractérisée en ce qu'**elle contient 1,8 à 4,2 mg de budésonide par suppositoire.

5. Formulation selon la revendication 4, **caractérisée en ce qu'**elle contient 1,8 à 2,2 mg de budésonide par suppositoire.

6. Formulation selon la revendication 4, **caractérisée en ce que** le poids par suppositoire se situe entre 3,8 et 4,2 mg de budésonide.

7. Formulation selon l'une quelconque des revendications précédentes, **caractérisée en ce que** le poids par suppositoire se situe entre 0,8 et 1,2 g.

8. Formulation selon l'une quelconque des revendications 1 à 7, **caractérisée en ce que** l'antioxydant est le palmitate d'ascorbyle.

9. Formulation selon la revendication 7, **caractérisée en ce que** le palmitate d'ascorbyle se présente en concentration de 50 ppm à 200 ppm.

10. Formulation selon l'une quelconque des revendications 1 à 9, **caractérisée en ce que** le budésonide se présente sous forme micronisée, dans laquelle 100 % des particules sont plus petites que 10 µm par particule.

11. Formulation selon l'une quelconque des revendications 1 à 10, **caractérisée en ce que** la formulation pharmaceutique stable au stockage est fabriquée en excluant l'oxygène.

12. Formulation selon l'une quelconque des revendications 1 à 11, **caractérisée en ce que** la formulation pharmaceutique est emballée sous forme de suppositoire dans une mince feuille moulée imperméable aux gaz.

13. Formulation pharmaceutique selon l'une quelconque des revendications 1 à 12 pour utilisation dans le traitement de maladies inflammatoires du rectum.

14. Formulation pharmaceutique selon l'une quelconque des revendications 1 à 12 pour utilisation dans le traitement d'une proctite ulcéro-hémorragique aiguë.

15. Formulation pharmaceutique pour utilisation selon l'une quelconque des revendications 13 ou 14 en combinaison avec des suppositoires de mésalazine.
